# EUROPEAN PATENT SPECIFICATION

(11) **EP 1 520 005 B1**
(45) Date of publication and mention of the grant of the patent: **12.09.2007**
(21) Application number: 03764300.4
(22) Date of filing: 23.05.2003
(51) Int. Cl.: C11D 17/04, C11D 3/00, C11D 3/382, C11D 3/386, A61K 8/02, A61K 8/64, C11D 3/38, A61K 8/66

(54) **CLEANERS FOR THE CONTROL AND REMOVAL OF ALLERGENS**
REINIGUNGSMITTEL ZUR STEUERUNG UND ENTFERNUNG VON ALLERGENEN
TAMPON DE NETTOYAGE POUR LE CONTROLE ET L'ELIMINATION D'ALLERGENES

(30) Priority: 10.07.2002 US 192053
(43) Date of publication of application: 06.04.2005
(73) Proprietor: KIMBERLY-CLARK WORLDWIDE, INC., Neenah, WI 54956 (US)
(72) Inventor: KOENIG, David, William, Menasha, WI 54952 (US); KISTLER, Annastacia, Appleton, WI 54911 (US); SCHMIDT, Richard, John, Roswell, GA 30076 (US); WILLIAMSON, Bruce, Scott, Alpharetta, GA 30005 (US)
(74) Representative: Beacham, Annabel Rose
(86) International application number: PCT/US2003/016398
(87) International publication number: WO 2004/007658

(56) References cited:
- BE-A- 1 003 561
- US-A- 5 407 454
- US-B1- 6 303 119
- PATENT ABSTRACTS OF JAPAN vol. 2002, no. 04, 4 August 2002 (2002-08-04) & JP 2001 348764 A (TSUBAKIMOTO CHAIN CO;OBAYASHI KANKYO GIJUTSU KENKYUSHO:KK; FINAL:KK), 21 December 2001 (2001-12-21)
- PATENT ABSTRACTS OF JAPAN vol. 2002, no. 10, 10 October 2002 (2002-10-10) -& JP 2002 167332 A (LION CORP), 11 June 2002 (2002-06-11)
- DATABASE WPI Section Ch, Week 199232 Derwent Publications Ltd., London, GB; Class B05, AN 1992-265109 XP002253055 -& JP 04 182423 A (SHISEIDO CO LTD) 30 June 1992 (1992-06-30) & PATENT ABSTRACTS OF JAPAN & JP 04 182423 A (SHISEIDO), 30 June 1992 (1992-06-30)

## Description

### Background of the Invention

The present invention relates to cleaners for removing allergens from various surfaces including human and animal skin and hair. More specifically, the present invention relates to cleaners comprising a wipe, such as a wet or dry wipe, comprising an additive comprising a lectin, a protease, and/or an enzyme inhibitor capable of binding an allergen, such as animal dander, to the cleaner and removing it from a surface. Additionally, the additive may be capable of reducing the allergenicity of the allergen upon contacting the allergen.

Allergens are substances that trigger allergic reactions in a host organism such as a human, and are typically small, acidic glycoproteins. Many allergens are members of a superfamily of extracellular proteins commonly referred to as lipocalins. Allergens such as animal dander and other animal secretions, such as saliva and urine, are commonplace in many households and businesses where animals and/or pets are present. Other allergens commonly found in the home include plant pollens, fungi allergens, cockroach allergens, and dust mite feces. These allergens are typically introduced into a host through direct inhalation of dust particles carrying the allergen into the nose and lungs, and can result in serious allergic reactions in the host such as perennial asthma, rhinitis, and conjunctivitis.

Animal dander, such as cat or dog dander for example, is one of the most common allergens. Animal dander is not only the hair or fur of the animal, but includes minute skin scales that fall from the hair, feathers, and skin of all warm-blooded animals. Animal dander carries antigens that can cause allergic reactions in sensitive people. Typically, older animals produce more skin-scale type dander than young ones because their skin is drier. Animal dander is extremely light weight and tiny in size, and can stay airborne for extended periods of time increasing the risk of penetration into the lower airways of exposed hosts. One specific cat allergen, Felis domesticus allergen I, has been shown to cause significant allergic reactions in some individuals. This allergen is a glycoprotein found in the sebaceous glands of the cat's hair roots and in their sublingual salivary glands and in the urine of male cats.

These and other allergens are commonly found on pets, humans, and various surfaces such as tables, furniture, and countertops. Common household cleaning methods and devices may be insufficient to substantially remove and/or neutralize many of the allergens commonly present on various surfaces. As such, a need exists for cleaners capable of substantially removing allergens such as pet dander from common household surfaces, as well as animals and human beings to decrease the amount of allergic reactions in hosts.

JP-A 2001 348764 discloses nonwoven cloths to which bark powder from a tree such as Thujopsis dolabrata, Chamaecyparis obtusa, Cryptomeria japonica or Eucalyptus globulus is bonded using a synthetic resin binder.

### Summary of the Invention

The present invention relates to cleaners comprising wet or dry wipes that can be used to remove allergens, such as animal dander and house dust mite feces, from various surfaces including human skin, animal coats and skin, and tables and countertops. The wipes utilized to form the cleaners of the present invention have immobilized thereon or therein an additive which is a lectin, optionally together with a protease, and/or an enzyme inhibitor, that can bind the allergen to the wipe substrate such that the allergen can be easily removed from the surface. When the cleaner comprises a protease or an enzyme inhibitor in combination with a lectin, the protease or enzyme inhibitor may provide the additional benefit of chemically altering the allergen to reduce its allergenicity. The additive-containing cleaners of the present invention are suitable for use on hard surfaces such as tables, countertops, and electronic boards, as well as on humans and animals to remove various allergens.

Briefly, therefore, the present invention is directed to a cleaner for removing an allergen from a surface, the cleaner comprising a wipe and an allergen binding lectin for binding the allergen to the wipe and removing the allergen from the surface, wherein the cleaner comprises from about 0.00001% to about 10% (by total weight of the treated cleaner) of the lectin, and wherein the wipe comprises a material selected from meltblown materials, coform materials, air-laid materials, bonded-carded web materials, hydroentangled materials, and combinations thereof.

The present invention is further directed to a method for removing an allergen from a surface, with the proviso that the surface is not a surface of human or animal body. The method comprises contacting the allergen on the surface with a cleaner according to the invention. The cleaner comprises a wipe and an allergen binding lectin for binding the allergen to the wipe and removing the allergen from the surface.

Other objects and features of this invention will be in part apparent and in part pointed out hereinafter.

### Detailed Description of the Preferred Embodiments

In accordance with the present invention, it has been discovered that cleaners comprising wet or dry wipes that have immobilized thereon or therein one or more additives that attract and bind numerous allergens, such as animal dander, to the wipe can be utilized to remove various allergens from hard surfaces. The immobilized additive comprises a lectin, and optionally also a protease, and/or an enzyme inhibitor, each of which will attract various allergens and bind to them thus allowing for removal of the allergen from a surface. Surprisingly, both proteases and enzyme inhibitor additives may provide the additional benefit of chemically reacting with the allergen to alter its chemistry and reduce its allergenicity in addition to binding the allergen. Cleaners treated in accordance with the present invention provide an economical and effective vehicle for removing numerous allergens from various surfaces to reduce the probability of the allergen causing an allergic response in a host such as a human being.

The additive-treated cleaners of the present invention are particularly suited for removing numerous allergens from various surfaces. As used herein, the term "allergen" is meant to include any antigen that is capable of inducing an allergic reaction in a host such as a human being. As mentioned above, many common allergens are lipocalins. Lipocalins are a large, diverse group of at least about 50 proteins that serve as transporters from small hydrophobic molecules such as lipids, steroid hormones, bilins, and retenoids. It has been speculated that with regard to animal allergens, lipocalins function biologically as pheromones or pheromone binding proteins.

Allergens which can be removed from surfaces in accordance with the present invention include, for example, animal allergens such as animal dander and animal saliva, plant allergens such as pollen, fungi, cockroach allergens, and house dust mite allergens including house dust mite feces. Specifically, the additive-treated wipes of the present invention are suited for removing allergens associated with domestic and laboratory animals such as dogs, cats, rabbits, mice rats and guinea pigs. More specifically, allergens such as Fel d 1, the most common cat allergen, and Can f 1 and Can f 2, the most common dog allergens can be removed from various surfaces utilizing the wipes of the present invention. Various common allergens are further detailed in Table 1.

**Table 1**

| Animal | Allergen | Molecular Weight | Source | Biological Function |
|---|---|---|---|---|
| Mouse | Mus m 1 | 19kD | Hair, dander, urine | Lipocalin-odorant binding protein |
| Mouse | Mus m 2 | 16kD | Hair, dander | Unknown |
| Mouse | Albumin | | Serum | Serum Protein |
| Rat | Rat n 1A | 16-21kD | Hair, dander, urine, saliva | Lipocalin-pheromone binding protein |
| Guinea pig | Cav p 1 | Unknown | Hair, dander, urine | Unknown |
| Guinea pig | Cav p 2 | Unknown | Hair, dander, urine | Unknown |
| Rabbit | Ory c 1 | 17kD | Hair, dander, saliva | Unknown |
| Rabbit | Ory c 2 | Unknown | Hair, dander, saliva | Unknown |
| Cat | Fel d 1 | 38kD | Hair, dander, saliva | Unknown |
| Cat | Albumin | | Serum | Serum Protein |
| Dog | Can f 1 | 25kD | Hair, dander, saliva | Lipocalin cystein protease inhibitor |
| Dog | Can f 2 | 19kD | Hair, dander, saliva | Lipocalin |
| Dog | Albumin | | Serum | Serum protein |

Based on the disclosure herein, one skilled in the art will recognize that the additive-treated wipes of the present invention will remove numerous allergens from various surfaces and the particular listing of allergens herein is merely illustrative and not meant to be in any way limiting.

The additives comprising a lectin as described herein are immobilized onto or incorporated into a wipe which is used to form the cleaners of the present invention. Additionally, any one or a combination of the additives could be introduced onto a furniture cover, such as a couch cover, to attract and potentially destroy allergens. Suitable wipes for forming the cleaners of the present invention include wet wipes, dry wipes, hand wipes, household wipes, industrial wipes, allergy wipes, and the like. Typically, conventional wipes have included a single layer of a substantially homogeneous material, but can also comprise a layered material comprised of more than one layer of homogeneous or heterogeneous material. Materials suitable for the substrate of the wipes described herein are well known to those skilled in the art, and are nonwoven fibrous sheet materials selected from meltblown, coform, air-laid, bonded-carded web materials, hydroentangled materials, and combinations thereof. Such materials can be comprised of synthetic or natural fibers, or a combination thereof. Typically, wipe substrates define a basis weight of from about 25 to about 120 grams per square meter and desirably from about 40 to about 90 grams per square meter.

In a particular embodiment, the wipe substrate incorporating the additives described herein comprise a coform basesheet of polymeric microfibers and cellulosic fibers having a basis weight of from about 60 to about 80 grams per square meter and desirably about 75 grams per square meter. Such coform basesheets are manufactured generally as described in U.S. Patent No. 4,100,324.

Typically, such coform basesheets comprise a gas-formed matrix of thermoplastic polymeric meltblown microfibers, such as, for example, polypropylene microfibers, and cellulosic fibers, such as, for example, wood pulp fibers.

The relative percentages of the polymeric microfibers and cellulosic fibers in the coform basesheet can vary over a wide range depending upon the desired characteristics of the wet wipes. For example, the coform basesheet may comprise from about 20 to about 100 weight percent, desirably from about 20 to about 60 weight percent, and more desirably from about 30 to about 40 weight percent of the polymeric microfibers based on the dry weight of the coform basesheet being used to provide the wet wipes.

Alternatively, the wipe substrates can comprise a composite which includes multiple layers of materials. For example, the wipe may include a three layer composite which includes an elastomeric film or meltblown layer between two coform layers as described above. In such a configuration, the coform layers may define a basis weight of from about 15 to about 30 grams per square meter and the elastomeric layer may include a film material such as a polyethylene metallocene film.

Each wipe is typically rectangular in shape and may have any suitable unfolded width and length. It will be recognized by one skilled in the art, however, that the shape, width, and length of the wipe is not critical to the present invention, and the wipes described herein can be any shape, width, and/or length suitable for the intended application. As an example, the wipe may have an unfolded length of from about 2.0 to about 80.0 centimeters and desirably from about 10.0 to about 25.0 centimeters and an unfolded width of from about 2.0 to about 80.0 centimeters and desirably from about 10.0 to about 25.0 centimeters. Typically, each individual wipe is arranged in a folded configuration and stacked one on top of the other to provide a stack of wipes. Such folded configurations are well known to those skilled in the art and include c-folded, z-folded, quarter-folded configurations and the like. The stack of folded wipes may be placed in the interior of a container, such as a plastic tub, to provide a package of wipes for eventual sale to a consumer. Alternatively, the wipes may include a continuous strip of material which has perforations between each wipe and which may be arranged in a stack or wound into a roll for dispensing.

In accordance with the present invention, the above-described wipes have one or more additives incorporated therein or thereon to facilitate the removal of allergens from a surface through the attraction to, or binding of, the allergen with the additive onto the wipe. The additive can be applied to the surface of the wipe or can be introduced into the fabric matrix either during or after the formation of the substrate. It is not critical whether the additive is on the surface of the wipe or impregnated into the fibers so long as the additive can contact or come into close proximity with the allergen during the use of the wipe.

In the present invention, the cleaner comprises a wipe having incorporated thereon or therein a lectin compound for attracting and binding allergens to allow removal of the allergens from a surface. As used herein, the term "lectin" or "lectin compound" is meant to include protein or glycoprotein compounds of plant or animal origin having a molecular weight of from about 60,000 Daltons to about 120,000 Daltons capable of specific recognition of and reversibly binding to, carbohydrate moieties of complex glycoconjugates such as allergens. Lectins are also commonly referred to as "phytohemagglutinins" or "phytagglutinins." Lectins typically chemically bind to sugar moieties of the allergen.

Without being bound to a particular theory, it is believed that the lectins used in combination with the cleaners described herein contact the allergen located on a surface as the cleaner is moved across the surface. Upon contacting the allergen, the lectin binds or bonds to one or more carbohydrate or sugar moieties located on the surface structure of the allergen such that the binding or bonding strength between the allergen and the lectin exceeds the forces retaining the allergen on the surface thereby allowing the allergen to be lifted from the surface onto the cleaner for removal. It is not believed that the lectins chemically interact substantially with the allergen to cleave numerous bonds to break the allergen into numerous peptides and lead to a significantly different structure or conformation, but simply bond onto the allergen to allow for its removal from the surface. The resulting bonding between the allergen and the lectin may be a covalent-type bonding.

Lectins suitable for use with the cleaners of the present invention include plant or animal lectins capable of binding or bonding to an allergen. Specifically, legume lectins are suitable for use in accordance with the present invention. More specifically, lectins having an affinity for N-acetylgalactosamine (GalNAc) residues are particularly suitable for incorporation into or onto the wipe substrates of the present invention for binding numerous allergens. It is preferred that the lectin be capable of binding or bonding to a lipocalin.

Specific examples of lectins suitable for use in accordance with the present invention include, but are not limited to, peanut agglutinin, Dolichus biflorus agglutinin, soy bean agglutinin, Lens culinaris agglutinin, Phaseolus agglutinin I, pealectin-I, Ricinus communis agglutinin, Jacalin lectins, Erythrina corallodendron, vicia Villosa, wheat germ agglutinin and combinations thereof. Particularly preferred lectins include peanut agglutinin, soybean agglutinin, and Jacalin lectins. One skilled in the art will recognize that numerous other plant and animal lectins could be used in accordance with the present invention to remove allergens from various surfaces.

In one embodiment of the present invention, lectins, which are proteins, can be utilized in combination with the cleaners of the present invention by introducing a lectin-containing protein extract into or onto the wipe comprising the cleaner. Because some lectins may be expensive to buy in purified form, or difficult to purchase in purified form, protein extracts from lectin plant or animal sources can be introduced into or onto the wipes without purifying out the lectin itself from the extract. Although not 100% purified lectins, the protein extracts of lectin sources typically contain a sufficiently high concentration of lectin to be highly useful in the present invention. For example, soy bean agglutinin can be introduced into a wipe by introducing a soy bean protein extract taken from a soy bean plant directly into or onto the wipe. The extract, although containing components other than lectins, also contains soybean agglutinin lectins in sufficient amounts as to provide the intended benefits of the present invention. As such, plant and animal protein extracts containing lectins may be a highly economical means of implementing the present invention.

Lectins can be combined with the cleaners in any amount suitable to bind allergens onto the cleaner for removal from a surface within the range of from about 0.00001% (by total weight of the treated cleaner) to about 10% (by total weight of the treated cleaner), preferably from about 0.00001% (by total weight of the treated cleaner) to about 1% (by total weight of the treated cleaner), and more preferably from about 0.00001% (by total weight of the treated cleaner) to about 0.1% (by total weight of the treated cleaner), As used herein, "by total weight of the treated cleaner" means the weight of the cleaner including the additive. For example, if the total weight of the cleaner is 10 grams and the additive had been incorporated at a level of 10% by the total weight of the cleaner, the cleaner prior to the addition of the additive would have weighed 9 grams and 1 gram of additive would have been added (1 gram is equal to 10% of the total weight of the cleaner).

In one embodiment of the present invention, a protease can also be introduced into or onto cleaner to bind and remove an allergen, such as animal dander, from a surface. As used herein, the term "protease" is meant to include any enzyme that catalyzes the hydrolysis of a protein during the first stage of its degradation to a simpler substance. The term "protease" may also be referred to in the art as a "protolytic enzyme" or as a "peptidase."

In this embodiment, the protease immobilized onto the cleaner will not only attract and bind the allergen to the cleaner to facilitate the removal of the allergen from the surface, but will provide the additional benefit of chemically reacting with the allergen to cleave bonds in the allergen to degrade the allergen into non-allergenic peptides to reduce the allergenicity of the allergen. The proteases described herein for immobilization onto the cleaner will chemically interact with the allergen and non-specifically attack and cleave bonds within the allergen structure thus disrupting the overall structure of the allergen and hence its ability to induce an allergic response in a host.

While it is believed that the protease immobilized into or onto the cleaner will typically bind the allergen onto the cleaner and allow the allergen to therefore be removed from a surface, the additional advantage of a chemical interaction between the protease and the allergen leading to the cleaving of various bonds and the rendering of the allergen less allergenistic may be important in those cases where the allergen actually removes the immobilized protease from the cleaner to the surface, or where the allergen, after being initially bound onto the cleaner is released back to the surface. In these cases, even though the allergen remains on the surface and not on the cleaner, the allergen, through chemical cleaving reactions with the protease, will be altered chemically and will be a less potent allergen and less likely to induce an allergic reaction in a host. Thus, the protease immobilized onto the substrate provides a dual benefit.

In accordance with this embodiment of the present invention, suitable proteases for immobilization onto the cleaner include both include both exopeptidases and endopeptidases. Preferably, the protease immobilized onto the cleaner is capable of chemically interacting with and cleaving bonds non-specifically (that is, at random and not at any specific or pre-determined sites) in lipocalins, a protein family to which numerous important allergen belong. Specific examples of suitable proteases include serine proteinases, cysteine proteinases, aspartic proteinases, and metallo proteinases. More specific examples of suitable proteases for use in combination with the cleaners discussed herein include chymotrypsin, trypsin, elastase, kallikrein subtilisin, papain, actinidin, bormelain, cathepsins, calpains, pepsin, chymosin, cathepsins, renin, penicillopepsin, rhizopuspepsin, endothiapepsin, thermolysin, neprilysin, aminopeptidase, and astacin. Particularly preferred proteases include endopeptidases such as bromelain, cathepsin B, cathepsin D, Cathepsin G, chymotrypsin, clostripain, collagenase, dispase, endoproteinase Arg-C, endoproteinase Asp-N, Endoproteinase Glu-C, Endoproteinase Lys-C, Factor Xa, Kallidrein, papain, pepsin, plasmin, proteinase K, subtilisin, thermolysin, thrombin, and trypsin.

Endopeptidases as set forth herein are preferred proteases for use in combination with the cleaners disclosed herein because they typically cleave or break apart a protein molecule by cleaving bonds throughout the molecular structure, and in particular in the middle or center of the structure as opposed to simply cleaving or breaking bonds on the ends of the chains. This type of cleaving action results in the protein being broken down into numerous smaller pieces of roughly the same molecular weight. On the other hand, exopeptidases, which are also useful in the present invention, typically cleave bonds from the ends of molecules and continue cleaving bonds toward the centers of molecules. As such, while an endopeptidase may break a 2000 unit protein molecule into several individual peptides having an average unit size of a few hundred units, an exopeptidase cleaving the same protein molecule will produce one peptide structure comprising 1900 units and several other peptides comprising only a few units resulting in less disruption of the allergen protein molecule.

Proteases can be combined with the cleaners in any amount suitable to bind allergens onto the cleaner to facilitate removal from a surface. Particularly preferred amounts of proteases to be combined with the cleaners are from about 0.0001% (by total weight of the treated cleaner) to about 10% (by total weight of the treated cleaner), more preferably from about 0.0001% (by total weight of the treated cleaner) to about 1% (by total weight of the treated cleaner), and still more preferably from about 0.0001% (by total weight of the treated cleaner) to about 0.1% (by total weight of the treated cleaner).

In another embodiment of the present invention, an enzyme inhibitor can also be introduced into or onto the cleaner to bind to, and remove allergens, such as animal dander, from a surface. As used herein, the term "enzyme inhibitor" is meant to include any compound or agent that combines with an enzyme in such a manner as to prevent the normal substrate-enzyme combination and the resulting catalytic reaction.

In this embodiment, the enzyme inhibitor immobilized onto the cleaner will not only bind onto allergens and remove them from a surface, but will also provide the additional benefit of chemically reacting with the allergen, or an enzyme required for the allergen to be allergenistic, to reduce the allergenicity of the allergen. Thus, the enzyme inhibitor interacts with the enzyme required by the allergen to be allergenistic and prevents the normal substrate-enzyme catalytic reaction from occurring such that the allergenicity of the allergen is significantly reduced or eliminated.

Without being bound to a particular theory, it is believed that allergens can exist chemically as proteins and/or as enzymes. When an allergen is a protein, it may require the enzymatic activity of a enzyme to render the allergen allergenistic, or at least to increase the level of allergenicity of the allergen. For example, an allergen may exist as a long chain protein which, by itself, because of its structure and chain size could not significantly penetrate the skin or mucosa membranes of a host and, therefore, would be expected to typically have a low allergenicity by itself. It is believed that this type of protein allergen structure becomes significantly more allergenistic when a specific protease enzyme interacts with the allergen to cleave the allergen at one or more specific bonding sites into smaller, allergenistically active, peptide fragments which can penetrate the skin or mucosa membrane of a host and lead to an allergic reaction. By introducing an enzyme inhibitor into or onto the cleaner and contacting the allergen and protease enzyme with the impregnated cleaner, the enzyme inhibitor will significantly reduce or inhibit the enzymatic activity of the protease enzyme resulting in a disruption of the enzyme-substrate interaction and a decrease in allergenicity.

In some cases it is believed that allergens may themselves exist as enzymes. In this case, the allergen can interact with itself to cleave specific bonds or otherwise chemically alter its structure to reduce the overall size or change the conformation of the allergen to allow penetration into skin or mucosa membrane openings. In this case, the allergen typically does not require an external protease enzyme to chemically alter its size or conformation to become allergenistic. By contacting the allergen, which exists as an enzyme, with an enzyme inhibitor, the allergenicity of the allergen is significantly reduced as the allergen cannot be specifically cleaved into smaller fragments or its conformation changed to allow for penetration into a host.

Without being bound to a particular theory, it is believed that the enzyme inhibitors immobilized onto the cleaners as set forth herein, will bind both protein-type and enzyme-type allergens onto the substrate allowing them to be removed from a surface upon contacting the wipe. With enzyme-type allergens, it is believed that the enzyme inhibitor is attracted to the enzyme-type allergen and a chemical interaction occurs whereby the enzymatic activity of the allergen is reduced; that is, a chemical interaction occurs wherein attraction and/or binding between the allergen and the enzyme inhibitor occurs allowing for the allergen to be attracted to and retained in the substrate of the wipe and removed from a surface. Regarding protein-type allergens, it is believed that the enzyme inhibitor chemically interacts with the required protease enzyme which may be in chemical contact with the protein and allow for attraction and binding of the entire complex onto the cleaner. Although it is believed that the enzyme inhibitors described herein will attract and bind for removal both enzyme-type allergens and protein-type allergen, it is further believed that the enzyme inhibitors are more likely to bind and retain enzyme-type allergens.

While it is believed that the enzyme inhibitor immobilized onto the cleaner will typically bind an allergen onto the cleaner and allow the allergen to therefore be removed from a surface, the additional advantage of having the enzyme inhibitor inhibit the enzymatic activity of the enzyme may be important in those cases where the allergen actually removes the immobilized enzyme inhibitor from the cleaner to the surface, or where the allergen, after being initially bound onto the cleaner is released back to the surface. In these cases, even though the allergen remains on the surface and not on the cleaner, the allergen, through the interaction with the enzyme inhibitor, may be a less potent allergen and less likely to induce an allergic reaction in a host. Thus, the enzyme inhibitors immobilized onto the web provide a dual benefit.

In accordance with this embodiment of the present invention, suitable enzyme inhibitors for immobilization on the cleaners of the present invention include, but are not limited to, Amastatin, Antipain, Actinonin, Aprotinin, APMSF, Bestatin, Benzamidine, Chymostatin, 3,4-dichloroisocoumarin, DFP, E-64, Elastatinal, Leupeptin, Pepstatin, 1,10-Phenanthroline, Phosphoramidon, TLCK, and TPCK.

Enzyme inhibitors can be combined with the wipe substrate in any amount suitable to bind allergens onto the cleaner to facilitate removal from a surface. Particularly preferred amounts of enzyme inhibitors to be combined with the cleaners are from about 0.0001% (by total weight of the treated cleaner) to about 10% (by total weight of the treated cleaner), more preferably from about 0.0001% (by total weight of the treated cleaner) to about 1% (by total weight of the treated cleaner), and still more preferably from about 0.0001% (by total weight of the treated cleaner) to about 0.1% (by total weight of the treated cleaner).

In an alternative embodiment of the present invention, the cleaners described herein can have immobilized thereon or therein a combination of lectins, and proteases and/or enzyme inhibitors to attract, bind and potentially reduce the allergenicity of, various allergens. In a preferred embodiment, the cleaner comprises a combination of lectins and proteases or a combination of lectins and enzyme inhibitors. Because it is believed that lectins simply attract and bind the allergen to the cleaner but do not significantly reduce the allergenicity of the allergen, it may be preferable to impregnate the cleaner with a protease and/or an enzyme inhibitor in addition to the lectin to act upon the bound allergen (or required enzyme) on the cleaner to ultimately reduce its allergenicity. When used in combination with the lectins, the protease or the enzyme inhibitor can chemically interact with the allergen to reduce its allergenicity on the substrate's surface. This may be important should the allergen be dislodged from the cleaner.

When lectins are used in combination with either proteases or enzyme inhibitors, the lectin is typically present in an amount of from about 0.0001% (by total weight of the treated cleaner) to about 10% (by total weight of the treated cleaner), more preferably from about 0.0001% (by total weight of the treated cleaner) to about 1% (by total weight of the treated cleaner), and still more preferably from about 0.0001% (by total weight of the treated cleaner) to about 0.1% (by total weight of the treated cleaner).

When proteases are used in combination with lectins, the protease is typically present in an amount of from about 0.0001% (by total weight of the treated cleaner) to about 10% (by total weight of the treated cleaner), more preferably from about 0.0001% (by total weight of the treated cleaner) to about 1% (by total weight of the treated cleaner), and still more preferably from about 0.0001% (by total weight of the treated cleaner) to about 0.1% (by total weight of the treated cleaner). Similarly, the enzyme inhibitors are used in combination with lectins, the enzyme inhibitor is typically present in an amount of from about 0.0001% (by total weight of the treated cleaner) to about 10% (by total weight of the treated cleaner), more preferably from about 0.0001% (by total weight of the treated cleaner) to about 1% (by total weight of the treated cleaner), and still more preferably from about 0.0001% (by total weight of the treated cleaner) to about 0.1% (by total weight of the treated cleaner).

The additives described herein can be immobilized or impregnated onto the surface of the cleaner by any means capable of stabilizing the additive on or near the cleaner's surface including, for example, printing, particle impingement and/or through the manipulation of electrostatic forces. In a preferred embodiment, the additive(s) is introduced onto or into a gas permeable cleaner in accordance with the present invention through the use of a vacuum driving force or through the use of a pressure differential. When utilizing a vacuum force, the additive(s) is positioned on the cleaner while a vacuum driving force is applied to the opposite side of the cleaner to drive the additive into the fabric matrix of the cleaner. Varying degrees of vacuum can be applied depending upon the required depth of the additive, and it is typically preferred that the additives remain at or near the surface of the cleaner for maximum effectiveness.

The additive-containing cleaners of the present invention can be utilized to remove allergens such as animal dander from a variety of surfaces including, for example, human skin, animal skin, human hair, animal hair, tabletops, countertops, electronic boards, and other hard or soft surfaces where allergens are problematic. The additive-containing cleaners described herein are particularly useful in removing animal or pet dander, such as cat or dog dander, from various surfaces in the home or office.

As mentioned herein, the additives can be immobilized on a cleaner to be used as a wet wipe or as a dry wipe. When a wet wipe is utilized, the wipe substrate will be in direct contact with a liquid, or wetting agent. The liquid formulation is not critical to the present invention with the exception that the components of the liquid portion of the wet wipe should not be substantially antagonistic to the additive(s) introduced onto the wipe substrate and cleaner. As such, the liquid can typically be any solution which can be absorbed into the wet wipe substrate and may include any suitable components which provide the desired wiping properties. For example, the components may include water, alcohol, emollients, surfactants, fragrances, preservatives, chelating agents, pH buffers or combinations thereof as are well known to those skilled in the art. Additionally, the liquid may contain lotions and/or medicaments.

In a less preferred embodiment of the present invention, the additives described herein can be introduced into the liquid component of a wet wipe system and be used in combination with a wet wipe substrate. In this embodiment, the additive is contained in the liquid component of the wet wipe system and, as such, may be retained on the surface being cleaned after wiping instead of being removed by the substrate. Although typically less desirable than binding, breaking apart, and removing the allergen from the surface, this embodiment is useful in some applications. For example, if the wet wipe solution comprises a lectin, the lectin may bind onto the allergen located on the surface and may thus inhibit the bound allergen from entering host and causing an allergic response because the combination of the lectin and the allergen it too large to effectively enter a host. Alternatively, if the solution comprises an enzyme inhibitor or a protease (or both), the allergen may be effectively neutralized and may not be capable of inducing an allergic reaction even if introduced into the host. One skilled in the art will also recognize that the additives described herein could also be introduced into a cleansing cream or lotion meant to be used to clean human and animal skin or hair. Similar to the additives in the wet wipe solutions described above, the additives in the cleansing cream or lotion may remain on the skin or hair, but could still provide significant benefits relating to the reduction of allergic responses.

The amount of liquid contained within each wet wipe may vary depending upon the type of material being used to provide the wet wipe, the type of liquid being used, the type of container being used to store the wet wipes, and the desired end use of the wet wipe. Generally, each wet wipe can contain from about 150 to about 600 weight percent and desirably from about 250 to about 450 weight percent liquid based on the dry weight of the wipe for improved wiping. In a particular aspect, the amount o of liquid contained within the wet wipe is from about 300 to about 400 weight percent and desirably about 330 weight percent based on the dry weight of the wet wipe. If the amount of liquid is less than the above-identified ranges, the wet wipe may be too dry and may not adequately perform. If the amount of liquid is greater than the above-identified ranges, the wet wipe may be oversaturated and soggy and the liquid may pool in the bottom of the container.

The present invention is illustrated by the following example which is merely for the purpose of illustration and is not to be regarded as limiting the scope of the invention or manner in which it may be practiced.

### Example 1

In this Example, various lectins were evaluated to determine whether the lectin exhibited a binding affinity for feline dander, a glycoprotein, at various feline dander concentrations.

Cat hair was obtained from two felines via routing grooming. Approximately 0.5 grams of fur from each cat was placed into the same 50 mL conical tube with 15 mL of Phosphate Buffered Saline (PBS). The tube was placed on a rocker overnight. The following day, the extracted dander was analyzed using the fel d 1 ELISA procedure referenced below. The extracted dander was tested neat, 1:10, 1:100, 1:1000, 1:10,000, 1:100,000, and 1:1,000,000. The dilutions were prepared in PBS.

The concentration of the extracted dander in the phosphate buffered saline solution was determined using a monoclonal Fel d 1 ELISA kit (Indoor Biotechnologies, Charlottesville, Va.).

The following lectins (bead bound, from Sigma Chemical St. Louis, Mo) were evaluated for their ability to bind feline dander: Concanavalin A, Jacalin, Peanut, Soybean, and UEA. Each lectin was separately introduced into 0.1 M Phosphate Buffered Saline (PBS) to produce a 0.4 milligram/milliliters solution of lectin in PBS. 500 microliters of the 0.4 milligram/milliliter lectin solution was introduced into a microcentrifuge tube. To the tube was added feline dander (500 microliters of either 3.4 microgram/milliliter dander in PBS or 37 micrograms/milliliter dander in PBS, depending on the experiment). The lectin/dander mixture was vortexed for 1 hour at room temperature to thoroughly mix the dander and lectin. Upon completion of the mixing, the lectin bound dander and free dander were separated by centrifugation (13,000 rpm for 5 minutes). The amount of free dander in the resulting supernate was determined using the Fel d 1 ELISA method referenced above. The amount of dander bound by the lectin is described by the ratio of the amount of free dander in lectin treated samples to the amount of free dander in control samples not exposed to the lectin.

As shown in Table 1, Jacalin, Peanut, and Soybean lectin all removed at least 50% of the added feline dander. Also, increasing the concentration of dander by about 10 fold did not appear to reduce the binding affinity of the Jacalin, Peanut, and Soybean lectins. Also, at the higher concentration, the Jacalin and Peanut (Soybean not tested at higher concentration) removed at least 90% of the dander in solution. Jacalin, Peanut, and Soybean lectins each have a residue affinity for N-acetyl-D-galactosamine while both Concanavalin A and UEA do not. This indicates that lectins having a residue affinity for N-acetyl-D-galactosamine are good candidates for dander binding.

**Table 1**

| Lectin | % Dander Bound (3.4 :g/mL) | % Dander Bound (37 :g/mL) |
|---|---|---|
| Concanavalin A | 52% | 0% |
| Jacalin | 72% | 95% |
| Peanut | 66% | 90% |
| Soybean | 58% | Not Done |
| UEA | -258% | 52% |

In view of the above, it will be seen that the several objects of the invention are achieved. As various changes could be made in the above-described additive-containing wipes without departing from the scope of the invention, it is intended that all matter contained in the above description be interpreted as illustrative and not in a limiting sense.

## Claims

1. A cleaner for removing an allergen from a surface, the cleaner comprising a wipe and an allergen binding lectin for binding the allergen to the wipe and removing the allergen from the surface, wherein the cleaner comprises from about 0.00001% to about 10% (by total weight of the treated cleaner) of the lectin, and wherein the wipe comprises a material selected from meltblown materials, coform materials, air-laid materials, bonded-carded web materials, hydroentangled materials, and combinations thereof.

2. The cleaner as set forth in claim 1 wherein the lectin has a residue affinity for N-acetylgalactosamine residues.

3. The cleaner as set forth in claim 1 wherein the lectin is selected from the group consisting of peanut agglutinin, Dolichus biflorus agglutinin, soy bean agglutinin, Lens culinaris agglutinin, Phaseolus agglutinin I, pealectin-I, Ricinus communis agglutinin, Jacalin, Erythrina corallodendron, vicia villosa, and wheat germ agglutinin.

4. The cleaner as set forth in claim 1 wherein the lectin is selected from the group consisting of peanut agglutinin, soy bean agglutinin, and Jacalin.

5. The cleaner as set forth in any one of claims 1 to 4 wherein the cleaner comprises from about 0.00001% to about 1% (by total weight of the treated cleaner) of the lectin.

6. The cleaner as set forth in any one of claims 1 to 4 wherein the cleaner comprises from about 0.00001% to about 0.1% (by total weight of the treated cleaner) of the lectin.

7. A cleaner as set forth in any preceding claim further comprising an allergen cleaving protease for cleaving the allergen to reduce the allergenicity of the allergen.

8. The cleaner as set forth in claim 7 wherein the protease is selected from the group consisting of exopeptidases and endopeptidases.

9. The cleaner as set forth in claim 7 or claim 8 wherein the protease is capable of chemically cleaving a lipocalin.

10. The cleaner as set forth in claim 9 wherein the protease is selected from the group consisting of serine proteinases, cystein proteinases, aspartic proteinases, and metallo proteinases.

11. The cleaner as set forth in claim 9 wherein the protease is selected from the group consisting of Chymotrypsin, Trypsin, Elastase, Kallikrein, Subtilisin, Papain, Actinidin, Bromelain, Cathepsins, Calpains, Pepsin, Chymosin, Cathepsins, Renin, Penicillopepsin, Rhizopuspepsin, Endothiapepsin, Thermolysin, Neprilysin, Aminopeptidase, Astacin, Cathepsin B, Cathepsin D, Cathepsin G, Clostripain, Collagenase, Dispase, Endoproteinase Arg-C, Endoproteinase Asp-N, Endoproteinase Glu-C, Endoproteinase Lys-C, Factor Xa, Plasmin, Proteinase K, and Thrombin.

12. The cleaner as set forth in claim 7 wherein the protease is selected from the group consisting of Bromelain, Cathespsin B, Cathepsin D, Cathepsin G, Chymotrypsin, Clostripain, Collagenase, Dispase, Endoproteinase Arg-C, Endoproteinase Asp-N, Endoproteinase Glu-C, Endoproteinase Lys-C, Factor Xa, Kallikrein, Papain, Pepsin, Plasmin, Proteinase K, Subtilisin, Thermolysin, Thrombin, and Trypsin.

13. The cleaner as set forth in any one of claims 7 to 12 wherein the cleaner comprises from about 0.0001% to about 10% (by total weight of the treated cleaner) of the protease.

14. A cleaner according to any one of claims 1 to 7, further comprising an enzyme inhibitor for chemically reacting with the allergen to reduce the allergenicity of the allergen.

15. The cleaner as set forth in claim 14 wherein the enzyme inhibitor is selected from the group consisting of Amastatin, Antipain, Actinonin, Aprotinin, APMSF, Bestatin, Benzamidine, Chymostatin, 3,4-dichloroisocoumarin, DFP, E-64, Elasatinal, Leupeptin, Pepstatin, 1,10-Phenanthroline, Phosphoramidon, TLCK, and TPCK.

16. The cleaner as set forth in claim 14 or claim 15 wherein the cleaner comprises from about 0.0001% to about 10% (by total weight of the treated cleaner) of the enzyme inhibitor.

17. The cleaner as set forth in any one of claims 1 to 16 wherein the wipe is comprised of fibers selected from the group consisting of cellulosic fibers, synthetic fibers, and combinations thereof.

18. A method for removing an allergen from a surface, the method comprising contacting the allergen on the surface with a cleaner according to any preceding claim, with the proviso that the surface is not a surface of a human or animal body.

## Patentansprüche

1. Reiniger zur Entfernung eines Allergens von einer Oberfläche, wobei der Reiniger einen Wischer und ein allergenbindendes Lektin zum Binden des Allergens an den Wischer und zur Entfernung des Allergens von der Oberfläche aufweist, wobei der Reiniger etwa 0,00001 % bis etwa 10 % (bezogen auf das Gesamtgewicht des behandelten Reinigers) des Lektins aufweist und wobei der Wischer ein Material aufweist, das unter schmelzgeblasenen Materialien, Coform-Materialien, Air-Laid-Materialien, Materialien für bondiert-kardierte Lagen, wasserstrahlverfestigten Materialien und Kombinationen daraus ausgewählt ist.

2. Reiniger nach Anspruch 1, wobei das Lektin eine Resteaffinität für N-Acetylgalactosaminreste hat.

3. Reiniger nach Anspruch 1, wobei das Lektin aus der Gruppe ausgewählt ist, die besteht aus Erdnuss-Agglutinin, Dolichus-biflorus-Agglutinin, Sojabohnen-Agglutinin, Lens-culinaris-Agglutinin, Phaseolus-Agglutinin I, Erbsenlektin-I, Ricinus-communis-Agglutinin, Jacalin, Erythrina corallodendron, Vicia villosa und Weizenkeim-Agglutinin.

4. Reiniger nach Anspruch 1, wobei das Lektin aus der Gruppe ausgewählt ist, die besteht aus Erdnuss-Agglutinin, Sojabohnen-Agglutinin und Jacalin.

5. Reiniger nach einem der Ansprüche 1 bis 4, wobei der Reiniger etwa 0,00001 % bis etwa 1 % (bezogen auf das Gesamtgewicht des behandelten Reinigers) des Lektins aufweist.

6. Reiniger nach einem der Ansprüche 1 bis 4, wobei der Reiniger etwa 0,00001 % bis etwa 0,1 % (bezogen auf das Gesamtgewicht des behandelten Reinigers) des Lektins aufweist.

7. Reiniger nach einem der vorhergehenden Ansprüche, der ferner eine allergenspaltende Protease zur Spaltung des Allergens zur Verringerung der Allergenität des Allergens aufweist.

8. Reiniger nach Anspruch 7, wobei die Protease aus der Gruppe ausgewählt ist, die besteht aus Exopeptidasen und Endopeptidasen.

9. Reiniger nach Anspruch 7 oder Anspruch 8, wobei die Protease zur chemischen Spaltung eines Lipocalins in der Lage ist.

10. Reiniger nach Anspruch 9, wobei die Protease aus der Gruppe ausgewählt ist, die besteht aus Serin-Proteinasen, Cystein-Proteinasen, Asparagin-Proteinasen und Metallo-Proteinasen.

11. Reiniger nach Anspruch 9, wobei die Protease aus der Gruppe ausgewählt ist, die besteht aus Chymotrypsin, Trypsin, Elastase, Kallikrein, Subtilisin, Papain, Actinidin, Bromelain, Cathepsinen, Calpainen, Pepsin, Chymosin, Renin, Penicillopepsin, Rhizopuspepsin, Endothiapepsin, Thermolysin, Neprilysin, Aminopeptidase, Astacin, Cathepsin B, Cathepsin D, Cathepsin G, Clostripain, Collagenase, Dispase, Endoproteinase Arg-C, Endoproteinase Asp-N, Endoproteinase Glu-C, Endoproteinase Lys-C, Faktor Xa, Plasmin, Proteinase K und Thrombin.

12. Reiniger nach Anspruch 7, wobei die Proteinase aus der Gruppe ausgewählt ist, die besteht aus Bromelain, Cathepsin B, Cathepsin D, Cathepsin G, Chymotrypsin, Clostripain, Collagenase, Dispase, Endoproteinase Arg-C, Endoproteinase Asp-N, Endoproteinase Glu-C, Endoproteinase Lys-C, Faktor Xa, Kallikrein, Papain, Pepsin, Plasmin, Proteinase K, Subtilisin, Thermolysin, Thrombin und Trypsin.

13. Reiniger nach einem der Ansprüche 7 bis 12, wobei der Reiniger etwa 0,0001 % bis etwa 10 % (bezogen auf das Gesamtgewicht des behandelten Reinigers) der Protease enthält.

14. Reiniger nach einem der Ansprüche 1 bis 7, der außerdem einen Enzyminhibitor zur chemischen Reaktion mit dem Allergen zur Verringerung der Allergenität des Allergens aufweist.

15. Reiniger nach Anspruch 14, wobei der Enzyminhibitor aus der Gruppe ausgewählt ist, die besteht aus Amastatin, Antipain, Actinonin, Aprotinin, APMSF, Bestatin, Benzamidin, Chymostatin, 3,4-Dichlorisocumarin, DFP, E-64, Elastatinal, Leupeptin, Pepstatin, 1,10-Phenanthrolin, Phosphoramidon, TLCK und TPCK.

16. Reiniger nach Anspruch 14 oder Anspruch 15, wobei der Reiniger etwa 0,0001 % bis etwa 10 % (bezogen auf das Gesamtgewicht des behandelten Reinigers) des Enzyminhibitors aufweist.

17. Reiniger nach einem der Ansprüche 1 bis 16, wobei der Wischer aus Fasern besteht, die ausgewählt sind aus der Gruppe, die besteht aus Cellulosefasern, synthetischen Fasern und Kombinationen daraus.

18. Verfahren zum Entfernen eines Allergens von einer Oberfläche, wobei das Verfahren das Kontaktieren des Allergens auf der Oberfläche mit einem Reiniger nach einem der vorstehenden Ansprüche umfasst, mit der Maßgabe, dass die Oberfläche nicht eine Oberfläche eines menschlichen oder tierischen Körpers ist.

## Revendications

1. Dispositif de nettoyage permettant d'éliminer un allergène d'une surface, le dispositif de nettoyage comprenant une lingette et une lectine se liant à l'allergène permettant de lier l'allergène à la lingette et d'éliminer l'allergène de la surface, **caractérisé en ce que** le dispositif de nettoyage comprend d'environ 0,00001 % à environ 10 % (en poids total du dispositif de nettoyage traité) de lectine et **en ce que** la lingette comprend un matériau choisi parmi les matériaux obtenus par fusion-soufflage, les matériaux de type "coform", les matériaux obtenus par un procédé par voie sèche aérodynamique, les matériaux en bandes cardées-liées, les matériaux hydroenchevêtrés et des combinaisons de ceux-ci.

2. Dispositif de nettoyage selon la revendication 1, **caractérisé en ce que** la lectine a une affinité de résidu pour les résidus N-acétylgalactosamine.

3. Dispositif de nettoyage selon la revendication 1, **caractérisé en ce que** la lectine est choisie dans le groupe constitué de l'agglutinine de cacahuète, de l'agglutinine de Dolichus biflorus, de l'agglutinine de soja, de l'agglutinine de Lens culinaris, de l'agglutinine I de Phaseolus, de la lectine I du pois, de l'agglutinine de Ricinus communis, de la jacaline, d'Erythrina corallodendron, de vicia villosa et de l'agglutinine de germe de blé.

4. Dispositif de nettoyage selon la revendication 1, **caractérisé en ce que** la lectine est choisie dans le groupe constitué de l'agglutinine de cacahuète, de l'agglutinine de soja et de la jacaline.

5. Dispositif de nettoyage selon l'une quelconque des revendications 1 à 4, **caractérisé en ce que** le dispositif de nettoyage comprend d'environ 0,00001 % à environ 1 % (en poids total du dispositif de nettoyage traité) de lectine.

6. Dispositif de nettoyage selon l'une quelconque des revendications 1 à 4, **caractérisé en ce que** le dispositif de nettoyage comprend d'environ 0,00001 % à environ 0,1 % (en poids total du dispositif de nettoyage traité) de lectine.

7. Dispositif de nettoyage selon l'une quelconque des revendications précédentes, comprenant en outre une protéase clivant l'allergène permettant de cliver l'allergène afin de réduire l'allergénicité de l'allergène.

8. Dispositif de nettoyage selon la revendication 7, **caractérisé en ce que** la protéase est choisie dans le groupe constitué des exopeptidases et des endopeptidases.

9. Dispositif de nettoyage selon la revendication 7 ou la revendication 8, **caractérisé en ce que** la protéase est susceptible de réaliser un clivage chimique d'une lipocaline.

10. Dispositif de nettoyage selon la revendication 9, **caractérisé en ce que** la protéase est choisie dans le groupe constitué des sérine protéinases, des cystéine protéinases, des protéinases aspartiques et des métalloprotéinases.

11. Dispositif de nettoyage selon la revendication 9, **caractérisé en ce que** la protéase est choisie dans le groupe constitué de la chymotrypsine, de la trypsine, de l'élastase, de la kallikréine, de la subtilisine, de la papaïne, de l'actinidine, de la bromélaïne, des cathépsines, des calpaïnes, de la pepsine, de la chymosine, de la rénine, de la pénicillopepsine, de la rhizopuspepsine, de l'endothiapepsine, de la thermolysine, de la néprilysine, de l'aminopeptidase, de l'astacine, de la cathepsine B, de la cathepsine D, de la cathepsine G, de la clostripaïne, de la collagénase, de la dispase, de l'endoprotéinase Arg-C, de l'endoprotéinase Asp-N, de l'endoprotéinase Glu-C, de l'endoprotéinase Lys-C, du facteur Xa, de la plasmine, de la protéinase K et de la thrombine.

12. Dispositif de nettoyage selon la revendication 7, **caractérisé en ce que** la protéase est choisie dans le groupe constitué de la bromélaïne, de la cathepsine B, de la cathepsine D, de la cathepsine G, de la chymotrypsine, de la clostripaïne, de la collagénase, de la dispase, de l'endoprotéinase Arg-C, de l'endoprotéinase Asp-N, de l'endoprotéinase Glu-C, de l'endoprotéinase Lys-C, du facteur Xa, de la kallikréine, de la papaïne, de la pepsine, de la plasmine, de la protéinase K, de la subtilisine, de la thermolysine, de la thrombine et de la trypsine.

13. Dispositif de nettoyage selon l'une quelconque des revendications 7 à 12, **caractérisé en ce que** le dispositif de nettoyage comprend d'environ 0,0001% à environ 10 % (en poids total du dispositif traité) de protéase.

14. Dispositif de nettoyage selon l'une quelconque des revendications 1 à 7, comprenant en outre un inhibiteur enzymatique destiné à réagir chimiquement avec l'allergène afin de réduire l'allergénicité de l'allergène.

15. Dispositif de nettoyage selon la revendication 14, **caractérisé en ce que** l'inhibiteur enzymatique est choisi dans le groupe constitué de l'amastatine, de l'antipaïne, de l'actinonine, de l'aprotinine, de l'APMSF, de la bestatine, de la benzamidine, de la chymostatine, de la 3,4-dichloroisocoumarine, du DFP, d'E-64, de l'élastatinal, de la leupeptine, de la pepstatine, de la 1,10-phénanthroline, du phosphoramidon, de la TLCK et de la TPCK.

16. Dispositif de nettoyage selon la revendication 14 ou la revendication 15, **caractérisé en ce que** le dispositif de nettoyage comprend d'environ 0,0001 % à environ 10 % (en poids total du dispositif de nettoyage traité) d'inhibiteur enzymatique.

17. Dispositif de nettoyage selon l'une quelconque des revendications 1 à 16, **caractérisé en ce que** la lingette est constituée de fibres choisies dans le groupe constitué des fibres cellulosiques, des fibres synthétiques et de combinaisons de celles-ci.

18. Procédé permettant d'éliminer un allergène d'une surface, le procédé comprenant la mise en contact de l'allergène sur la surface avec un dispositif de nettoyage selon l'une quelconque des revendications précédentes, à condition que la surface ne soit pas une surface d'un corps humain ou animal.
